# EUROPEAN PATENT SPECIFICATION

(11) **EP 1 478 755 B1**
(45) Date of publication and mention of the grant of the patent: **27.05.2009**
(21) Application number: 03704338.7
(22) Date of filing: 18.02.2003
(51) Int. Cl.: C12N 15/67, C12N 15/80, C12N 15/65

(54) **EXPRESSION CLONING METHODS IN FILAMENTOUS FUNGI**
EXPRESSIONSKLONIERUNGSVERFAHREN IN FILAMENTÖSEN PILZEN
PROCEDES DE CLONAGE D'EXPRESSION DANS DES CHAMPIGNONS FILAMENTEUX

(30) Priority: 19.02.2002 DK 200200256
(43) Date of publication of application: 24.11.2004
(73) Proprietor: Novozymes A/S, 2880 Bagsvaerd (DK)
(72) Inventor: STRINGER, Mary, Ann, DK-2100 Copenhagen O (DK); SCHNORR, Kirk, DK-2840 Holte (DK); VIND, Jesper, DK-3500 Vaerlose (DK)
(86) International application number: PCT/DK2003/000106
(87) International publication number: WO 2003/070956

(56) References cited:
- WO-A-00/24883
- WO-A-01/51646
- WO-A-89/01969
- WO-A-94/11523
- US-A- 6 107 477

### SEQUENCE LISTING

<110> Novozymes A/S
   Stringer, Mary
   Schnorr, Kirk
   Vind, Jesper
<120> Expression cloning methods in filamentous fungi
<130> 10164.204-WO
<160> 20
<170> PatentIn version 3.1
<210> 1
   <211> 10
   <212> DNA
   <213> Artificial sequence
<220>
   <223> crippled consensus Kozak sequence
   <220>
   <221> misc_feature
   <222> (1)..(10)
   <223> n denotes any nucleotide, and Y denotes a Pyrimidin (cytidine or Thymidine/uridine.
<400> 1
   nynnatgynn 10
<210> 2
   <211> 30
   <212> DNA
   <213> Artificial sequence
<220>
   <223> Primer 141200J1
<400> 2
   atcggtttta tgtcttccaa gtcgcaattg 30
<210> 3
   <211> 33
   <212> DNA
   <213> Artificial sequence
<220>
   <223> Primer 141200J2
<400> 3
   cttggaagac ataaaaccga tggaggggta gcg 33
<210> 4
   <211> 26
   <212> DNA
   <213> Artificial sequence
<220>
   <223> Primer 270999J8
<400> 4
   tctgtgaggc ctatggatct cagaac 26
<210> 5
   <211> 27
   <212> DNA
   <213> Artificial sequence
<220>
   <223> Primer 270999J9
<400> 5
   gatgctgcat gcacaactgc acctcag 27
<210> 6
   <211> 59
   <212> DNA
   <213> Artificial sequence
<220>
   <223> Primer 051199J1
<400> 6
   cctctagatc tcgagctcgg tcaccggtgg cctccgcggc cgctggatcc ccagttgtg 59
<210> 7
   <211> 33
   <212> DNA
   <213> Artificial sequence
<220>
   <223> Primer 1298TAKA
<400> 7
   gcaagcgcgc gcaatacatg gtgttttgat cat 33
<210> 8
   <211> 31
   <212> DNA
   <213> Artificial sequence
<220>
   <223> Primer 142779
<400> 8
   ttgaattgaa aatagattga tttaaaactt c 31
<210> 9
   <211> 25
   <212> DNA
   <213> Artificial sequence
<220>
   <223> Primer 142780
<400> 9
   ttgcatgcgt aatcatggtc atagc 25
<210> 10
   <211> 26
   <212> DNA
   <213> Artificial sequence
<220>
   <223> Primer 140288
<400> 10
   ttgaattcat gggtaataac tgatat 26
<210> 11
   <211> 32
   <212> DNA
   <213> Artificial sequence
<220>
   <223> Primer 142778
<400> 11
   aaatcaatct attttcaatt caattcatca tt 32
<210> 12
   <211> 45
   <212> DNA
   <213> Artificial sequence
<220>
   <223> Primer 141223
<400> 12
   ggatgctgtt gactccggaa atttaacggt ttggtcttgc atccc 45
<210> 13
   <211> 44
   <212> DNA
   <213> Artificial sequence
<220>
   <223> Primer 141222
<400> 13
   ggtattgtcc tgcagacggc aatttaacgg cttctgcgaa tcgc 44
<210> 14
   <211> 27
   <212> DNA
   <213> Artificial sequence
<220>
   <223> Primer P50 - 260301j1
<220>
   <221> misc_feature
   <222> (1)..(27)
   <223> n denotes any nucleotide.
<400> 14
   acaggactcg gtncgtacat tgccgtg 27
<210> 15
   <211> 27
   <212> DNA
   <213> Artificial sequence
<220>
   <223> Primer F91 - 260301j2
<220>
   <221> misc_feature
   <222> (1)..(27)
   <223> n denotes any nucleotide.
<400> 15
   aatttcctca tctncgaaga tcgcaag 27
<210> 16
   <211> 27
   <212> DNA
   <213> Artificial sequence
<220>
   <223> Primer F96 - 260301j3
<220>
   <221> misc_feature
   <222> (1)..(27)
   <223> n denotes any nucleotide.
<400> 16
   gaagatcgca agtncatcga tatcgga 27
<210> 17
   <211> 30
   <212> DNA
   <213> Artificial sequence
<220>
   <223> Primer N101,T102 - 260301j4
<220>
   <221> misc_feature
   <222> (1)..(30)
   <223> n denotes any nucleotide.
<400> 17
   atcgatatcg ganacancgt ccaaaagcag 30
<210> 18
   <211> 27
   <212> DNA
   <213> Artificial sequence
<220>
   <223> Primer G128 - 26030lj5
<220>
   <221> misc_feature
   <222> (1)..(27)
   <223> n denotes any nucleotide.
<400> 18
   agtattctgc ccgntgaggg tatcgtc 27
<210> 19
   <211> 30
   <212> DNA
   <213> Artificial sequence
<220>
   <223> Primer G222, D223 - 260301j6
<220>
   <221> misc_feature
   <222> (1)..(30)
   <223> n denotes any nucleotide.
<400> 19
   ctctcctcga aggntnacaa gctgggacag 30
<210> 20
   <211> 27
   <212> DNA
   <213> Artificial sequence
<220>
   <223> Primer G239 - 230301j7
<220>
   <221> misc_feature
   <222> (1)..(27)
   <223> n denotes any nucleotide.
<400> 20
   gctgttggac gcgntgccga ctttatt 27

## Description

### Background of the invention

Several methods for the construction of libraries of polynucleotide sequences of interest in yeast have been disclosed in which the libraries are screened in yeast prior to transformation of an industrially relevant filamentous fungal host cell with a selected polynucleotide.

Often however, a polynucleotide sequence identified by screening in yeast or bacteria cannot be expressed or is expressed at low levels when transformed into production relevant filamentous fungal cells. This may be due any number of reasons, including differences in codon usage, regulation of mRNA levels, translocation apparatus, post-translational modification machinery (e.g., cysteine bridges, glycosylation and acylation patterns), etc.

A. Aleksenko and A.J. Clutterbuck (1997. Fungal Genetics and Biology 21:373-387) disclose the use of autonomous replicative vectors, or autonomously replicating sequences (ARS), for gene cloning and expression studies. AMA1 (autonomous maintenance in *Aspergillus*) is one of the plasmid replicator elements discussed. It consists of two inverted copies of a genomic repeat designated MATE1 (mobile *Aspergillus* transformation enhancer) separated by a 0.3 kb central spacer. AMA1 promotes plasmid replication without rearrangement, multimerization or chromosomal integration. AMA1-based plasmids provide two advantages in gene cloning in filamentous fungi. The first is a high frequency of transformation which both increases the potential library size and can eliminate the need for library amplification in an intermediate host, e.g., E. coli, so that a recipient *Aspergillus* strain can be transformed directly with a ligation mixture. Secondly, by providing a stable and standard environment for gene expression, the properties of the transformants will be uniform (WO 00/24883; Novozymes A/S).

Kozak, 1981, Nucleic Acids Research 9: 5233-5252, proposed the following "consensus" sequence for initiation of translation in higher eukaryotes:
Aa Acc aug G
In this sequence, often referred to as a "consensus Kozak", the most highly conserved nucleotides are the purines, adenine(A) and guanine (G), shown in capital letters above; the start-codon of the gene to be translated is underlined in the above. Mutational analysis confirmed that these two positions have the strongest influence on initiation (Kozak, 1987, Molecular Cell Biology 7: 3438-3445). Kozak also determined that alterations in the sequence upstream of the consensus Kozak can effect translation (Kozak, 1986, Proceedings of the National Academy of Sciences USA 83: 2850-2854).

WO 94/11523 and WO 01/51646 disclose expression vectors comprising a fully impaired consensus Kozak or "crippled" consensus Kozak sequence.

### Summary of the Invention

Expression cloning as such in filamentous fungi is presently part of the standard methodology in the art, however the use of such methods is of such industrial relevance that even minor increments in efficiency, performance or economy is of great interest. Until now expression cloning in filamentous fungi may have provided an interesting polypeptide candidate, whereupon the encoding gene would typically have been sub-cloned into a more suitable expression vector to achieve polypeptide yields of sufficient quantity to further characterize the polypeptide of interest, before setting up expensive larger scale trial productions. A problem to be solved is how to screen a polynucleotide library for a polypeptide with a property of interest in a filamentous fungal host cell in a manner which allows quick and easy characterization of the subsequent polypeptide.

An aspect of the present invention relates to methods for isolating a recombinant polypeptide of interest, the methods comprising the steps of:
a) providing a polynucleotide library derived from an organism capable of producing one or more polypeptides of interest, wherein the library was prepared in an expression cloning vector comprising at least the following elements:
   i) a polynucleotide encoding a selectable marker in which the translation initiation start site of the marker-encoding sequence comprises the following sequence: 
-4
N YNN ATG YNN (SEQ ID NO:1)
 wherein "Y" in position -3 is a pyrimidin (Cytidine or Thymidine/Uridine), "N" is any nucleotide, and the numerical designations are relative to the first nucleotide in the start-codon "ATG" (in bold) of the marker;
   ii) a fungal replication initiation sequence, preferably an automously replicating sequence (ARS), more preferably an AMA1-sequence or a functional derivative thereof; and
   iii) a polynucleotide comprising in sequential order: a promoter derived from a filementous fungal cell, a cloning-site into which the library is cloned, and a transcription terminator;
b) transforming a filamentous fungal host cell with the library;
c) culturing the transformed host cell obtained in (b) under conditions suitable for expression of the polynucleotide library; and
d) selecting a transformed host cell which produces the polypeptide of interest.

### Detailed Description of the Invention

The present invention relates to a method of the first aspect of the invention for isolating a recombinant polypeptide of interest, the method comprising the steps of:
a) providing a polynucleotide library derived from an organism capable of producing one or more polypeptides of interest, wherein the library was prepared in an expression cloning vector comprising at least the following elements:
   i) a polynucleotide encoding a selectable marker in which the translation initiation start site of the marker-encoding sequence comprises the following sequence: 
-4
N YNN ATG YNN (SEQ ID NO:1)
 wherein "Y" in position -3 is a pyrimidin (Cytidine or Thymidine/Uridine), "N" is any nucleotide, and the numerical designations are relative to the first nucleotide in the start-codon "ATG" (in bold) of the marker;
   ii) a fungal replication initiation sequence, preferably an automously replicating sequence (ARS), more preferably an AMA1-sequence or a functional derivative thereof; and
   iii) a polynucleotide comprising in sequential order: a promoter derived from a filementous fungal cell, a cloning-site into which the library is cloned, and a transcription terminator;
b) transforming a filamentous fungal host cell with the library;
c) culturing the transformed host cell obtained in (b) under conditions suitable for expression of the polynucleotide library; and
d) selecting a transformed host cell which produces the polypeptide of interest.

In the production methods of the present invention, the cells are cultivated in a nutrient medium suitable for production of the polypeptide, and under conditions that select for multiple copies of the selectable marker, using methods known in the art. For example, the cell may be cultivated by shake flask cultivation, or small-scale or large-scale fermentation (including continuous, batch, fed-batch, or solid state fermentations) in laboratory or industrial fermentors performed in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (*e.g*., in catalogues of the American Type Culture Collection).

If the polypeptide of interest is secreted into the nutrient medium, the polypeptide can be recovered directly from the medium. If the polypeptide is not secreted, it can be recovered from cell lysates.

The polypeptide may be detected using methods known in the art that are specific for the polypeptides. These detection methods may include use of specific antibodies, formation of an enzyme product, or disappearance of an enzyme substrate. The polypeptide may be recovered by methods known in the art. For example, the polypeptide may be recovered from the nutrient medium by conventional procedures including, but not limited to, centrifugation, filtration, extraction, spray-drying, evaporation, or precipitation.

The polypeptides may be purified by a variety of procedures known in the art including, but not limited to, chromatography (*e.g*., ion exchange, affinity, hydrophobic, chromatofocusing, and size exclusion), electrophoretic procedures (*e.g*., preparative isoelectric focusing), differential solubility (*e.g*., ammonium sulfate precipitation), SDS-PAGE, or extraction (see, *e.g.,* Protein Purification, J.-C. Janson and Lars Ryden, editors, VCH Publishers, New York, 1989).

### Crippled Translational Initiator Sequences

The term "translational initiator sequence" is defined herein as the ten nucleotides immediately upstream of the initiator or start codon of the open reading frame of a polypeptide-encoding nucleic acid sequence. The initiator codon encodes for the amino acid methionine, the so-called "start" codon. The initiator codon is typically an ATG, but may also be any functional start codon such as GTG. It is well known in the art that uracil (uridine), U, replaces the deoxynucleotide thymine (thymidine), T, in RNA.

The term "crippled translational initiator sequence" is defined herein as the ten nucleotides immediately upstream of the initiator codon of the open reading frame of a polypeptide-encoding nucleic acid sequence, wherein the initiator sequence comprises a T at the -3 position and a T at one or more of the -1, -2, and -4 positions.

Accordingly, a preferred embodiment of the invention relates to a method of the first aspect, wherein the sequence SEQ ID NO:1 comprises a Thymidin (Uridin) in the -3 position; even more preferably the the sequence SEQ ID NO:1 further comprises a Thymidin (Uridin) in one more of the positions -1, -2, and -4.

The term "operably linked" is defined herein as a configuration in which a control sequence, *e.g*., a crippled translational initiator sequence, is appropriately placed at a position relative to a coding sequence such that the control sequence directs the production of a polypeptide encoded by the coding sequence.

The term "coding sequence" is defined herein as a nucleic acid sequence that is transcribed into mRNA which is translated into a polypeptide when placed under the control of the appropriate control sequences. The boundaries of the coding sequence are generally determined by the start codon located at the beginning of the open reading frame of the 5' end of the mRNA and a stop codon located at the 3' end of the open reading frame of the mRNA. A coding sequence can include, but is not limited to, genomic DNA, cDNA, semisynthetic, synthetic, and recombinant nucleic acid sequences.

In the methods of the present invention, the crippled translational initiator sequence is foreign to the gene encoding a selectable marker.

The crippled translational sequence results in inefficient translation of the gene encoding the selectable marker. When a fungal host cell harbouring an expression vector comprising a polynucleotide encoding a polypeptide of interest physically linked with a second polynucleotide comprising a crippled translational initiator sequence operably linked to a gene encoding a selectable marker, is cultured under conditions that select for multiple copies of the selectable marker, the copy number of the polypeptide-encoding polynucleotide cloned into the vector is also increased.

The term "selectable marker" is defined herein as a gene the product of which provides for biocide or viral resistance, resistance to heavy metals, prototrophy to auxotrophs, and the like, which permits easy selection of transformed cells. Selectable markers for use in a filamentous fungal host cell include, but are not limited to, *amdS* (acetamidase), *argB* (omithine carbamoyltransferase), bar (phosphinothricin acetyltransferase), *hygB* (hygromycin phosphotransferase), *niaD* (nitrate reductase), *pyrG* (orotidine-5'-phosphate decarboxylase), *sC* (sulfate adenyltransferase), *trpC* (anthranilate synthase), as well as equivalents thereof. Preferred for use in an *Aspergillus* cell are the *amdS* and *pyrG* genes of *Aspergillus nidulans* or *Aspergillus oryzae* and the *bar* gene of *Streptomyces hygroscopicus.* Functional derivatives of these selectable markers are also of interest in the present invention, in particular those functional derivatives which have decreased activity or decreased stability, thereby enabling a selection for a higher copy-number of the expression vector without increasing the concentration of the selective substance(s).

Accordingly, a preferred embodiment is a method of the first aspect, wherein the selectable marker of step (i) is selected from the group of markers consisting of *amdS, argB, bar, hygB, niaD, pyrG, sC, and trpC;* preferably the selectable marker of step (i) is *pyrG* or a functional derivative thereof, more preferably the selectable marker of step (i) is a functional derivative of *pyrG* which comprises a substitution of one or more amino acids, and most preferably the derivative comprises the amino acid substitution T102N.

The term "copy number" is defined herein as the number of molecules, per genome, of a gene which is contained in a cell. Methods for determining the copy number of a gene are will known in the art and include Southern analysis, quantitative PCR, or real time PCR.

The fungal host cell preferably contains at least two copies, more preferably at least ten copies, even more preferably at least one hundred copies, most preferably at least five hundred copies, and even most preferably at least one thousand copies of the expression cloning vector.

### Polypeptide encoding polynucleotides

The polypeptide of interest may be native or heterologous to the filamentous fungal host cell of interest. The term "heterologous polypeptide" is defined herein as a polypeptide which is not native to the fungal cell, a native polypeptide in which modifications have been made to alter the native sequence, or a native polypeptide whose expression is quantitatively altered as a result of a manipulation of the fungal cell by recombinant DNA techniques. The polynucleotide encoding the polypeptide of interest may originate from any organism capable of producing the polypeptide of interest, including multicellular organisms and microorganisms e.g. bacteria and fungi.

A preferred embodiment of the invention relates to methods of the first aspect, wherein the organism of step (a) capable of producing one or more polypeptides of interest is a eukaryote, preferably the eukaryote is a fungus, and most preferably a filamentous fungus.

The term "polypeptide" is not meant herein to refer to a specific length of the encoded product and, therefore, encompasses peptides, oligopeptides, and proteins.

Preferably, the polypeptide of interest is an enzyme, an enzyme variant, or a functional derivative thereof, more preferably the enzyme or enzyme variant is an oxidoreductase, transferase, hydrolase, lyase, isomerase, or ligase; and most preferably the enzyme or enzyme variant is an aminopeptidase, amylase, carbohydrase, carboxypeptidase, catalase, cellulase, chitinase, cutinase, cyclodextrin glycosyltransferase, deoxyribonuclease, esterase, alpha-galactosidase, beta-galactosidase, glucoamylase, alpha-glucosidase, beta-glucosidase, invertase, laccase, lipase, mannosidase, mutanase, oxidase, a pectinolytic enzyme, peroxidase, phytase, polyphenoloxidase, proteolytic enzyme, ribonuclease, transglutaminase, or xylanase.

Preferably, the polypeptide is a hormone or hormone variant or a functional derivative thereof, a receptor or receptor variant or a functional derivative thereof, an antibody or antibody variant or a functional derivative thereof, or a reporter.

In a preferred embodiment, the polypeptide is secreted extracellularly. In a more preferred embodiment, the polypeptide is an oxidoreductase, transferase, hydrolase, lyase, isomerase, or ligase. In an even more preferred embodiment, the polypeptide is an aminopeptidase, amylase, carbohydrase, carboxypeptidase, catalase, cellulase, chitinase, cutinase, cyclodextrin glycosyltransferase, deoxyribonuclease, esterase, alpha-galactosidase, beta-galactosidase, glucoamylase, alpha-glucosidase, beta-glucosidase, invertase, laccase, lipase, mannosidase, mutanase, oxidase, pectinolytic enzyme, peroxidase, phospholipase, phytase, polyphenoloxidase, proteolytic enzyme, ribonuclease, transglutaminase, or xylanase.

The nucleic acid sequence encoding a polypeptide of interest may be obtained from any prokaryotic, eukaryotic, or other source. For purposes of the present invention, the term "obtained from" as used herein in connection with a given source shall mean that the polypeptide is produced by the source or by a cell in which a gene from the source has been inserted.

The techniques used to isolate or clone a nucleic acid sequence encoding a polypeptide of interest are known in the art and include isolation from genomic DNA, preparation from cDNA, or a combination thereof. The cloning of the nucleic acid sequence from such genomic DNA can be effected, *e.g*., by using the well known polymerase chain reaction (PCR). See, for example, Innis et al., 1990, PCR Protocols: A Guide to Methods and Application, Academic Press, New York. The cloning procedures may involve excision and isolation of a desired nucleic acid fragment comprising the nucleic acid sequence encoding the polypeptide, insertion of the fragment into a vector molecule, and incorporation of the recombinant vector into the mutant fungal cell where multiple copies or clones of the nucleic acid sequence will be replicated. The nucleic acid sequence may be of genomic, cDNA, RNA, semisynthetic, synthetic origin, or any combinations thereof.

In the methods of the present invention, the polypeptide may also include a fused or hybrid polypeptide in which another polypeptide is fused at the N-terminus or the C-terminus of the polypeptide or fragment thereof. A fused polypeptide is produced by fusing a nucleic acid sequence (or a portion thereof) encoding one polypeptide to a nucleic acid sequence (or a portion thereof) encoding another polypeptide. Techniques for producing fusion polypeptides are known in the art, and include, ligating the coding sequences encoding the polypeptides so that they are in frame and expression of the fused polypeptide is under control of the same promoter(s) and terminator. The hybrid polypeptide may comprise a combination of partial or complete polypeptide sequences obtained from at least two different polypeptides wherein one or more may be heterologous to the mutant fungal cell.

Once a transformed host cell has been selected which produces the polypeptide of interest according to the methods of the invention, the encoding polynucleotide can be isolated from the selected transformed host cell, and a further optimized expression system can be designed.

Accordingly, a preferred embodiment relates to methods of the first aspect, wherein subsequently to step (d) the polynucleotide coding for the polypeptide of interest is isolated from the selected transformed host cell of step (d).

### Fungal replication initiating sequences

As used herein, the term "fungal replication initiating sequence" is defined as a nucleic acid sequence which is capable of supporting autonomous replication of an extrachromosomal molecule, e.g., a DNA vector such as a plasmid, in a filamentous fungal host cell, normally without structural rearrangement of the DNA-vector or integration into the host cell genome. The replication initiating sequence may be of any origin as long as it is capable of mediating replication intiating activity in a fungal cell. For instance the replication initiating sequence may be a telomer of human origin which confer to the plasmid the ability to replicate in Aspergillus (Aleksenko and Ivanova, Mol.Gen. Genet. 260 (1998) 159-164). Preferably, the replication initiating sequence is obtained from a filamentous fungal cell, more preferably a strain of *Aspergillus, Fusarium* or *Alternaria,* and even more preferably, a strain of *A. nidulans, A. oryzae, A. niger, F. oxysporum* or *Alternaria altenata.*

A fungal replication initiating sequence may be identified by methods well-known in the art. For instance, the sequence may be identified among genomic fragments derived from the organism in question as a sequence capable of sustaining autonomous replication in yeast, (Ballance and Turner, Gene, 36 (1985), 321-331), an indication of a capability of autonomous replication in filamentous fungal cells. The replication initiating activity in fungi of a given sequence may also be determined by transforming fungi with contemplated plasmid replicators and selecting for colonies having an irregular morphology, indicating loss of a sectorial plasmid which in turn would lead to lack of growth on selective medium when selecting for a gene found on the plasmid (Gems et al, Gene, 98 (1991) 61-67). AMA1 was isolated in this way. An alternative way to isolate a replication initiating sequence is to isolate natural occurring plasmids (eg as disclosed by Tsuge et al., Genetics 146 (1997) 111-120 for *Alternaria aternata).*

Examples of fungal replication initiating sequences include, but are not limited to, the ANS1 and AMA1 sequences of *Aspergillus nidulans,* e.g., as described, respectively, by Cullen, D., et al. (1987, Nucleic Acids Res. 15:9163-9175) and Gems, D., et al. (1991, Gene 98:61-67).

Preferred embodiments relate to methods of the first aspect of the invention, wherein the fungal replication initiation sequence of step (ii) comprises the nucleic acid sequence set forth in SEQ ID NO:1 or SEQ ID NO:2 of WO 00/24883, or is a functional derivative thereof, preferably the functional derivative is at least 80% identical to SEQ lD NO:1 or SEQ ID NO: 2 of WO 00/24883.

The term "replication initiating activity" is used herein in its conventional meaning, i.e. to indicate that the sequence is capable of supporting autonomous replication of an extrachromosomal molecule, such as a plasmid or a DNA vector in a fungal cell.

The term "without structural rearrangement of the plasmid" is used herein to mean that no part of the plasmid is deleted or inserted into another part of the plasmid, nor is any host genomic DNA inserted into the plasmid. The replication initiating sequence to be used in the methods of the present invention is a nucleotide sequence having at least 50% identity with the nucleic acid sequence of SEQ ID NO:1 or SEQ ID NO:2 of WO 00/24883, and is capable of initiating replication in a fungal cell; or a subsequence of (a) or (b), wherein the subsequence is capable of initiating replication in a fungal cell.

In a preferred embodiment, the nucleotide sequence has a degree of identity to the nucleic acid sequence shown in SEQ ID NO:1 or SEQ ID NO:2 of WO 00/24883 of at least 50%, more preferably at least 60%, even more preferably at least 70%, even more preferably at least 80%, even more preferably at least 90%, and most preferably at least 97% identity (hereinafter "homologous polynucleotide"). The homologous polynucleotide also encompasses a subsequence of SEQ ID NO:1 or SEQ ID NO:2 of WO 00/24883 which has replication initiating activity in fungal cells. For purposes of the present invention, the degree of identity may be suitably determined by means of computer programs known in the art, such as GAP provided in the GCG program package (Program Manual for the Wisconsin Package, Version 8, August 1994, Genetics Computer Group, 575 Science Drive, Madison, Wisconsin, USA 53711) (Needleman, S.B. and Wunsch, C.D., (1970), Journal of Molecular Biology, 48, 443-45), using GAP with the following settings for polynucleotide sequence comparison: GAP creation penalty of 5.0 and GAP extension penalty of 0.3.

The techniques used to isolate or clone a nucleic acid sequence having replication initiating activity are known in the art and include isolation from genomic DNA or cDNA. The cloning from such DNA can be effected, *e.g*., by using methods based on polymerase chain reaction (PCR) to detect cloned DNA fragments with shared structural features. (See, *e.g*., Innis, et al., 1990, PCR: A Guide to Methods and Application, Academic Press, New York.) Other nucleic acid amplification procedures such as ligase chain reaction (LCR) may be used.

In preferred embodiment, the replication initiating sequence has the nucleic acid sequence set forth in SEQ ID NO:1 or SEQ ID NO:2 of WO 00/24883, or a respective functional subsequence thereof. For instance, a functional subsequence of SEQ ID NO:1 of WO 00/24883 is a nucleic acid sequence encompassed by SEQ ID NO:1 or SEQ ID NO 2 of WO 00/24883 except that one or more nucleotides from the 5' and/or 3' end have been deleted. Preferably, a subsequence contains at least 100 nucleotides, more preferably at least 1000 nucleotides, and most preferably at least 2000 nucleotides. In a more preferred embodiment, a subsequence of SEQ ID NO:1 of WO 00/24883 contains at least the nucleic acid sequence shown in SEQ ID NO:2 of WO 00/24883.

### Nucleic Acid Constructs

The present invention also relates to nucleic acid constructs comprising a polynucleotide comprising a crippled translational initiator sequence operably linked to a gene encoding a selectable marker in which the 3' end of the crippled translational initiator sequence is immediately upstream of the initiator codon of the gene encoding the selectable marker. The polynucleotides are operably linked to one or more control sequences which direct the expression of the coding sequence in a suitable host cell under conditions compatible with the control sequences. Expression will be understood to include any step involved in the production of the polypeptide including, but not limited to, transcription, post-transcriptional modification, translation, post-translational modification, and secretion.

"Nucleic acid construct" is defined herein as a nucleic acid molecule, either single-or double-stranded, which is isolated from a naturally occurring gene or which has been modified to contain segments of nucleic acid combined and juxtaposed in a manner that would not otherwise exist in nature. The term nucleic acid construct is synonymous with the term expression vector when the nucleic acid construct comprises a second polynucleotide encoding a polypeptide of interest and all the control sequences required for its expression.

An isolated polynucleotide encoding a polypeptide may be further manipulated in a variety of ways to provide for expression of the polypeptide. Manipulation of the nucleic acid sequence prior to its insertion into a vector may be desirable or necessary depending on the expression vector. The techniques for modifying nucleic acid sequences utilizing recombinant DNA methods are well known in the art.

In the methods of the present invention, the nucleic acid sequences may comprise one or more native control sequences or one or more of the native control sequences may be replaced with one or more control sequences foreign to the nucleic acid sequence for improving expression of the coding sequence in a host cell.

The term "control sequences" is defined herein to include all components which are necessary or advantageous for the expression of a polypeptide of interest. Each control sequence may be native or foreign to the nucleic acid sequence encoding the polypeptide. Such control sequences include, but are not limited to, a leader, polyadenylation sequence, propeptide sequence, crippled translational initiator sequence of the present invention, signal peptide sequence, and transcription terminator. At a minimum, the control sequences include translational initiator sequences, and transcriptional and translational stop signals. The control sequences may be provided with linkers for the purpose of introducing specific restriction sites or cloning sites facilitating ligation of the control sequences with the coding region of the nucleic acid sequence encoding a polypeptide.

The control sequence may be an appropriate promoter sequence, a nucleic acid sequence which is recognized by a host cell for expression of the nucleic acid sequence. The promoter sequence contains transcriptional control sequences which mediate the expression of the polypeptide. The promoter may be any nucleic acid sequence which shows transcriptional activity in the host cell of choice including mutant, truncated, and hybrid promoters, and may be obtained from genes encoding extracellular or intracellular polypeptides either homologous or heterologous to the host cell.

Examples of suitable promoters for directing the transcription of the nucleic acid constructs of the present invention in a filamentous fungal host cell are promoters obtained from the genes for *Aspergillus oryzae* TAKA amylase, *Rhizomucor miehei* aspartic proteinase, *Aspergillus niger* neutral alpha-amylase, *Aspergillus niger* acid stable alpha-amylase, *Aspergillus niger* or *Aspergillus awamori* glucoamylase *(glaA), Rhizomucor miehei* lipase, *Aspergillus oryzae* alkaline protease, *Aspergillus oryzae* triose phosphate isomerase, *Aspergillus nidulans* acetamidase, *Fusarium venenatum* amyloglucosidase, *Fusarium oxysporum* trypsin-like protease (WO 96/00787), as well as the NA2-tpi promoter (a hybrid of the promoters from the genes for *Aspergillus niger* neutral alpha-amylase and *Aspergillus oryzae* triose phosphate isomerase); and mutant, truncated, and hybrid promoters thereof.

A preferred embodiment relates to methods of the first aspect, wherein the promoter of step (iii) is the promoter from the neutral amylase encoding gene (NA2) from *Aspergillus niger* disclosed in WO 89/01969.

The control sequence may be a suitable transcription terminator sequence, a sequence recognized by a host cell to terminate transcription. The terminator sequence is operably linked to the 3' terminus of the nucleic acid sequence encoding the polypeptide. Any terminator which is functional in the host cell of choice may be used in the present invention.

Preferred terminators for filamentous fungal host cells are obtained from the genes for *Aspergillus oryzae* TAKA amylase, *Aspergillus niger* glucoamylase, *Aspergillus nidulans* anthranilate synthase, *Aspergillus niger* alpha-glucosidase, and *Fusarium oxysporum* trypsin-like protease.

A preferred embodiment relates to methods of the first aspect, wherein the transcription terminator of step (iii) is the terminator from the glucoamylase encoding gene (AMG) from *Aspergillus niger* (Boel,E.; Hjort,I.; Svensson,B.; Norris,F.; Norris,K.E.; Fiil,N.P., Glucoamylases G1 and G2 from Aspergillus niger are synthesized from two different but closely related mRNAs. EMBO J. 3:1097 (1984)).

The control sequence may also be a suitable leader sequence, a nontranslated region of an mRNA which is important for translation by the host cell. The leader sequence is operably linked to the 5' terminus of the nucleic acid sequence encoding the polypeptide. Any leader sequence that is functional in the host cell of choice may be used in the present invention.

Preferred leaders for filamentous fungal host cells are obtained from the genes for *Aspergillus oryzae* TAKA amylase and *Aspergillus nidulans* triose phosphate isomerase.

A preferred embodiment relates to methods of the first aspect, wherein the promoter is operably linked, upstream of the cloning-site of step (iii), to the polynucleotide encoding the leader peptide of triose phosphate isomerase (tpiA) from *Aspergillus nidulans.* (Mcknight G.L., O'Hara P.J., Parker M.L.,"Nucleotide sequence of the triosephosphate isomerase gene from Aspergillus nidulans: Implications for a differential loss of introns",Cell 46:143-147(1986)).

The control sequence may also be a polyadenylation sequence, a sequence operably linked to the 3' terminus of the nucleic acid sequence and which, when transcribed, is recognized by the host cell as a signal to add polyadenosine residues to transcribed mRNA. Any polyadenylation sequence which is functional in the host cell of choice may be used in the present invention.

Preferred polyadenylation sequences for filamentous fungal host cells are obtained from the genes for *Aspergillus oryzae* TAKA amylase, *Aspergillus niger glucoamylase, Aspergillus nidulans* anthranilate synthase, *Fusarium oxysporum* trypsin-like protease, and *Aspergillus niger* alpha-glucosidase.

The control sequence may also be a signal peptide coding region that codes for an amino acid sequence linked to the amino terminus of a polypeptide and directs the encoded polypeptide into the cell's secretory pathway. The 5' end of the coding sequence of the nucleic acid sequence may inherently contain a signal peptide coding region naturally linked in translation reading frame with the segment of the coding region which encodes the secreted polypeptide. Alternatively, the 5' end of the coding sequence may contain a signal peptide coding region which is foreign to the coding sequence. The foreign signal peptide coding region may be required where the coding sequence does not naturally contain a signal peptide coding region. Alternatively, the foreign signal peptide coding region may simply replace the natural signal peptide coding region in order to enhance secretion of the polypeptide. However, any signal peptide coding region which directs the expressed polypeptide into the secretory pathway of a host cell of choice may be used in the present invention.

Effective signal peptide coding regions for filamentous fungal host cells are the signal peptide coding regions obtained from the genes for *Aspergillus oryzae* TAKA amylase, *Aspergillus niger* neutral amylase, *Aspergillus niger* glucoamylase, *Rhizomucor miehei* aspartic proteinase, *Humicola insolens* cellulase, and *Humicola lanuginosa* lipase.

The control sequence may also be a propeptide coding region that codes for an amino acid sequence positioned at the amino terminus of a polypeptide. The resultant polypeptide is known as a proenzyme or propolypeptide (or a zymogen in some cases). A propolypeptide is generally inactive and can be converted to a mature active polypeptide by catalytic or autocatalytic cleavage of the propeptide from the propolypeptide. The propeptide coding region may be obtained from the genes for *Bacillus subtilis* alkaline protease (*aprE*), *Bacillus subtilis* neutral protease (*nprT*)*, Saccharomyces cerevisiae* alpha-factor, *Rhizomucor miehei* aspartic proteinase, and *Myceliophthora thermophila* laccase (WO 95/33836).

Where both signal peptide and propeptide regions are present at the amino terminus of a polypeptide, the propeptide region is positioned next to the amino terminus of a polypeptide and the signal peptide region is positioned next to the amino terminus of the propeptide region.

### Expression Vectors

The present invention also relates to recombinant expression vectors comprising a crippled translational initiator sequence operably linked to a gene encoding a selectable marker in which the 3' end of the crippled translational initiator sequence is immediately upstream of the initiator codon of the gene encoding the selectable marker and a nucleic acid sequence encoding a polypeptide of interest as well as any control sequences involved in the expression of the sequences.

The various nucleic acid and control sequences described above may be joined together to produce a recombinant expression vector which may include one or more convenient restriction sites to allow for insertion or substitution of the promoter and/or nucleic acid sequence encoding the polypeptide at such sites. Alternatively, the nucleic acid sequence may be expressed by inserting the nucleic acid sequence or a nucleic acid construct comprising the crippled translational initiator sequence and/or sequence into an appropriate vector for expression. In creating the expression vector, the coding sequence is located in the vector so that the coding sequence is operably linked with a crippled translational initiator sequence of the present invention and one or more appropriate control sequences for expression.

The recombinant expression vector may be any vector (*e.g*., a plasmid or virus) which can be conveniently subjected to recombinant DNA procedures and can bring about the expression of a nucleic acid sequence. The choice of the vector will typically depend on the compatibility of the vector with the host cell into which the vector is to be introduced. The vectors may be linear or closed circular plasmids.

The vector may be an autonomously replicating vector, *i.e.*, a vector which exists as an extrachromosomal entity, the replication of which is independent of chromosomal replication, *e.g*., a plasmid, an extrachromosomal element, a minichromosome, or an artificial chromosome. The vector may contain any means for assuring self-replication.

The vectors of the present invention also contain one or more selectable markers which permit easy selection of transformed cells as described earlier.

For autonomous replication, the vector further comprises an origin of replication enabling the vector to replicate autonomously in the host cell in question. Examples of origins of replication for use in a yeast host cell are the 2 micron origin of replication, ARS1, ARS4, the combination of ARS1 and CEN3, and the combination of ARS4 and CEN6. The origin of replication may be one having a mutation which makes its functioning temperature-sensitive in the host cell (see, *e.g*., Ehrlich, 1978, Proceedings of the National Academy of Sciences USA 75: 1433).

The procedures used to ligate the elements described above to construct the recombinant expression vectors of the present invention are well known to one skilled in the art (see, *e.g*., Sambrook *et al.,* 1989, *supra).*

### Host Cells

The host cell may be any fungal cell useful in the methods of the present invention. "Fungi" as used herein includes the phyla Ascomycota, Basidiomycota, Chytridiomycota, and Zygomycota (as defined by Hawksworth et al., In, Ainsworth and Bisby's Dictionary of The Fungi, 8th edition, 1995, CAB International, University Press, Cambridge, UK) as well as the Oomycota (as cited in Hawksworth *et al.,* 1995, *supra,* page 171) and all mitosporic fungi (Hawksworth *et a*/*.,* 1995, *supra*)*.*

In a preferred embodiment, the fungal host cell is a filamentous fungal cell. "Filamentous fungi" include all filamentous forms of the subdivision Eumycota and Oomycota (as defined by Hawksworth *et al*., 1995, *supra*)*.* The filamentous fungi are characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

In a preferred embodiment, the filamentous fungal host cell is a cell of a species of, but not limited to, *Acremonium, Aspergillus, Fusarium, Humicola, Mucor, Myceliophthora, Neurospora, Penicillium, Thielavia, Tolypocladium,* or *Trichoderma.*

In a more preferred embodiment, the filamentous fungal host cell is an *Aspergillus awamori, Aspergillus foetidus, Aspergillus japonicus, Aspergillus nidulans, Aspergillus niger* or *Aspergillus oryzae* cell. In another most preferred embodiment, the filamentous fungal host cell is a *Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides,* or *Fusarium venenatum* cell. In another most preferred embodiment, the filamentous fungal host cell is a *Humicola insolens, Humicola lanuginosa, Mucor miehei, Myceliophthora thermophila, Neurospora crassa, Penicillium purpurogenum, Thielavia terrestris, Trichoderma harzianum, Trichoderma koningii, Trichoderma longibrachiatum, Trichoderma reesei,* or *Trichoderma viride* cell.

In an even most preferred embodiment, the *Fusarium venenatum* cell is *Fusarium venenatum* A3/5, which was originally deposited as *Fusarium graminearum* ATCC 20334 and recently reclassified as *Fusarium venenatum* by Yoder and Christianson, 1998, Fungal Genetics and Biology 23: 62-80 and O'Donnell et al., 1998, Fungal Genetics and Biology 23: 57-67; as well as taxonomic equivalents of *Fusarium venenatum* regardless of the species name by which they are currently known. In another preferred embodiment, the *Fusarium venenatum* cell is a morphological mutant of *Fusarium venenatum* A3/5 or *Fusarium venenatum* ATCC 20334, as disclosed in WO 97/26330.

Fungal cells may be transformed by a process involving protoplast formation, transformation of the protoplasts, and regeneration of the cell wall in a manner known *per se.* suitable procedures for transformation of *Aspergillus* host cells are described in EP 238 023 and Yelton et al., 1984, Proceedings of the National Academy of Sciences USA 81: 1470-1474. Suitable methods for transforming *Fusarium* species are described by Malardier et al., 1989, Gene 78: 147-156 and WO 96/00787.

The present invention is further described by the following examples which should not be construed as limiting the scope of the invention.

### Examples

### Example 1

In order to improve expression of a gene of interest on an expression plasmid, it may be desirable to reduce the expression of the selection gene, exemplified here by the *pyrG* gene. By cultivating a host cell harbouring an expression plasmid comprising a selection gene, that has reduced expression, under normal selective pressure results in a selection for a host cell which has an increased plasmid copy number, thus achieving the total expression level of the selection gene necessary for survival. The higher plasmid copy-number however also results in an increased expression of the gene of interest.

One way of decreasing the expression level of the selection gene is to lower the mRNA level by either using a poorly transcribed promoter or decreasing the functional halflife of the mRNA. Another way is to reduce translation efficiency of the mRNA. One way to do this is to mutate the Kozak-region. This is a region just upstream of the initiation codon (ATG), which is important for the initiation of translation.

**Plasmid pENI2155** comprises a bad kozak region upstream of the *pyrG* gene, and is constructed as follows:
Using plasmid pENI1861 (the construction of which is described below) as template, and PWO polymerase (conditions as recommended by manufacturer); two PCR-reactions were made using primer 141200j1 and 270999J9 in the one PCR-reaction and primers 141200J2 and 290999J8 in another PCR-reaction:
   141200J1 (SEQ ID NO:2): 5' atcggttttatgtcttccaagtcgcaattg
   141200J2 (SEQ ID NO:3): 5' cttggaagacataaaaccgatggaggggtagcg
   270999J8 (SEQ ID NO:4): 5' tctgtgaggcctatggatctcagaac
   270999J9 (SEQ ID NO:5): 5' gatgctgcatgcacaactgcacctcag

The PCR fragments were purified from a 1% agarose gel using QIAGEN™ spin columns. A second PCR-reaction was run using the two fragments as template along with the primers 270999J8 and 270999J9. The PCR-fragment from this reaction was purified from a 1% agarose gel as described; the fragment and the vector pENI1849 (containing a lipase gene as expression reporter) were cut with the restriction enzymes Stul and Sphl, the resulting fragments were purified from a 1% agarose gel as described previously.

The purified fragments were ligated and transformed into the *E.coli* strain DH10B. Plasmid DNA from one of the transformants was isolated and sequenced to confirm the introduction of a mutated Kozak region: ggtttt**atg** (rather than the wildtype: gccaac**atg**). This Plasmid was denoted: pENl2155.

*Aspergillus* cells were transformed with plasmid pENi1849 (control wildtype plasmid), and pENi2155 (mutated Kozak region upstream of the *pyrG* gene). Approximately 1 microgram of pENI11849 and pENi2155 were transformed into *A. oryzae* Jal355 (JaL355 is a derivative of *A. oryzae* A1560 wherein the *pyrG* gene has been inactivated, as described in WO 98/01470; transformation protocol as described in WO 00/24883). The transformants were incubated for 4 days at 37°C.

24 transformants from the pENi2155 transformation and 12 transformants from pENI1849 were inoculated in a 96 well microtiter plate containing 1*Vogel medium and 2 % maltose (Methods in Enzymology, vol. 17, p. 84). After 4 days growth at 34°C, the culture broth was assayed for lipase activity using pnp-valerate as a lipase substrate.

A 10 microliter aliquot of media from each well was added to a microtiter well containing 200 microliter of a lipase substrate of 0.018% p-nitrophenylvalerate, 0.1 % Triton X™-100, 10 mM CaCl₂, 50 mM Tris pH 7.5. Lipase activity was assayed spectrophotometrically at 15-second intervals over a five minute period, using a kinetic microplate reader (Molecular Device Corp., Sunnyvale CA), using a standard enzymology protocol (e.g., Enzyme Kinetics, Paul C.Engel, ed., 1981, Chapman and Hall Ltd.). Briefly, product formation is measured during the initial rate of substrate turnover and is defined as the slope of the curve calculated from the absorbance at 405 nm every 15 seconds for 5 minutes. The arbitrary lipase activity units were normalized against the transformant showing the highest lipase activity. For each group of thirty transformants an average value and the standard deviations were calculated. Given in arbitrary units the average lipase activity and relative standard deviation was:
1849 Transformant: 65 ± 14
2155 Transformant: 120 ± 22

Clearly there is nearly a doubling of lipase expression in the 2155 transformant, wherein the mutated Kozak region was introduced in front of the selection gene *pyrG.*

**Plasmid pENI1861** was made in order to have the state of the art *Aspergillus* promoter in the expression plasmid, as well as a number of unique restriction sites for cloning. A PCR fragment (Approx. 620 bp) was made using plasmid pMT2188 (the construction of pMT2188 is described below) as template and the following primers:
051199J1 (SEQ ID NO:6): 5' cctctagatctcgagctcggtcaccggtggcctccgcggccgctggatccccagttgtg
1298TAKA (SEQ ID NO:7): 5' gcaagcgcgcgcaatacatggtgttttgatcat

The fragment was cut with Bss*H*II and *Bgl*II, and cloned into pENI1849 which was also cut with BssHII and Bgl II. The cloning was verified by sequencing.

**Plasmid pENI1849** was made in order to truncate the pyrG gene to the essential sequences for pyrG expression, in order to decrease the size of the plasmid, thus improving transformation frequency. A PCR fragment (Approx. 1800 bp) was made using pENI1299 (described in WO 00/24883 Fig. 2 and Example 1) as template and the following primers: 270999J8 (SEQ ID NO:3), and 270999J9 (SEQ ID NO:4)

The PCR-fragment was cut with the restriction enzymes Stul and Sphl, and cloned into pENI1298 (described in WO 00/24883 Fig. 1 and Example 1), also cut with Stul and Sphl; the cloning was verified by sequencing.

**Plasmid pMT2188** was based on the *Aspergillus* expression plasmid pCaHj 483 (described in WO 98/00529) which consists of an expression cassette based on the *Aspergillus niger* neutral amylase II promoter fused to the *Aspergillus nidulans* triose phosphate isomerase non translated leader sequence (Pna2/tpi) and the *A*. *niger* amyloglycosidase terminater (Tamg). Also present on the pCaHj483 is the *Aspergillus* selective marker *amdS* from *A. nidulans* enabling growth on acetamide as sole nitrogen source. These elements are cloned into the *E. coli vector* pUC19 (New England Biolabs). The ampicillin resistance marker enabling selection in *E. coli* of pUC19 was replaced with the URA3 marker of *Saccharomyces cerevisiae* that can complement a *pyrF* mutation in *E. coli,* the replacement was done in the following way:

The pUC19 origin of replication was PCR amplified from pCaHj483 with the primers:
142779 (SEQ ID NO:8): 5' ttgaattgaaaatagattgatttaaaacttc
142780 (SEQ ID NO:9): 5' ttgcatgcgtaatcatggtcatagc

Primer 142780 introduces a *Bbu*I site in the PCR fragment. The Expand™ PCR system (Roche Molecular Biochemicals, Basel, Switserland) was used for the amplification following the manufacturers instructions for this and the subsequent PCR amplifications.

The URA3 gene was amplified from the general *S. cerevisiae* cloning vector pYES2 (Invitrogen corporation, Carlsbad, Ca, USA) using the primers:
140288 (SEQ ID NO:10): 5' ttgaattcatgggtaataactgatat
142778 (SEQ ID NO:11): 5' aaatcaatctattttcaattcaattcatcatt

Primer 140288 introduces an *Eco*RI site in the PCR fragment. The two PCR fragments were fused by mixing them and amplifying using the primers 142780 and 140288 in the splicing by overlap method (Horton et al (1989) Gene, 77, 61-68).

The resulting fragment was digested with *Eco*RI and *Bbu*I and ligated to the largest fragment of pCaHj 483 digested with the same enzymes. The ligation mixture was used to transform the *pyrF E.coli* strain DB6507 (ATCC 35673) made competent by the method of Mandel and Higa (Mandel, M. and A. Higa (1970) J. Mol. Biol. 45, 154). Transformants were selected on solid M9 medium (Sambrook et. al (1989) Molecular cloning, a laboratory manual, 2. edition, Cold Spring Harbor Laboratory Press) supplemented with 1 g/l casaminoacids, 500 microgram/l thiamine and 10 mg/l kanamycin.

A plasmid from a selected transformant was termed pCaHj527. ThePna2/tpi promoter present on pCaHj527 was subjected to site directed mutagenises by a simple PCR approach. Nucleotide 134-144 was altered from GTACTAAAACC to CCGTTAAATTT using the mutagenic primer 141223. Nucleotide 423-436 was altered from ATGCAATTTAAACT to CGGCAATTTAACGG using the mutagenic primer 141222. The resulting plasmid was termed **pMT2188.**
Primer 141223 (SEQ ID NO:12): 5' ggatgctgttgactccggaaatttaacggtttggtcttgcatccc
Primer 141222 (SEQ ID NO:13): 5' ggtattgtcctgcagacggcaatttaacggcttctgcgaatcgc

### Example 2

In order to improve expression of a gene of interest from a plasmid, it may be desirable to reduce the stability and/or the activity of the protein encoded by the selection gene (for instance the *pyrG* gene) as already mentioned in Example 1.

One way of decreasing the stability of the protein encoded by the selection gene is to add a "degron" motif to the protein (Dohmen R.J., Wu P., Varshavsky A., (1994) Science vol 263 p. 1273-1276). Another way is to identify structurally important conserved amino acid residues, based on alignment to homologous proteins or based on a model-structure of the protein (if available). These amino acids may then be mutated to decrease the stability and/or the activity of the enzyme.

A protein alignment was made with the protein sequence: swissprot_dcop_aspng (the OMP decarboxylase encoded by the pyrG gene on plasmid pENI2155) to the following database entries: Swissprot_dcop-aspor, geneseqp_r05224, geneseqp_y99702, tremblnew_aag34761, swissprot_dcop_phyb), remtermbl_aab01165, remtembl_aab16845, and sptrembl_q9uvz5.

The alignment was done using the program ClustalW (Thompson, J.D., Higgins, D.G. and Gibson, T.J. (1994) CLUSTAL W: improving the sensitivity of progressive multiple sequence alignment through sequence weighting, positions-specific gap penalties and weight matrix choice. Nucleic Acids Research, 22:4673-4680).

Based on these alignments and the structure of the related *Bacillus subtilis* OMP decarboxylase (Appleby t., Kinsland C., Begley T.P., Ealick S.E.. (2000), Proc. Natl. Acad. Sci. USA, vol 97 p. 2005-2010) the following conserved residues were identified as potentially structurally important, and as such suitable targets for mutation: P50, F91, F96, N101, T102, G128, G222, D223, G239. A number of mutagenic primers were constructed, and were phosphorylated using T4 polynucleotide kinase (New England Biolabs).
P50 - 260301j1 (SEQ ID NO:14): 5' acaggactcggtncgtacattgccgtg
F91 - 260301 j2 (SEQ ID NO:15): 5' aatttcctcatctncgaagatcgcaag
F96 - 260301 j3 (SEQ ID NO:16): 5' gaagatcgcaagtncatcgatatcgga
N101,T102 - 260301j4 (SEQ ID NO:17): 5' atcgatatcgganacancgtccaaaagcag
G128 - 260301j5 (SEQ ID NO:18): 5' agtattctgcccgntgagggtatcgtc
G222, D223 - 260301j6 (SEQ ID NO:19): 5' ctctcctcgaaggntnacaagctgggacag
G239 - 230301j7 (SEQ ID NO:20): 5' gctgttggacgcgntgccgactttatt

Seven individual PCR/ligation reactions were performed (as described by Sawano A., Miyawaki A. (2000) Nucleic Acid Research vol 28 e78) using pENl2155 as template, and 1 microliter DNA from each of the seven libraries was transformed into the *E. coli* strain DH10B. Approximately 1000 *E. coli clones* were obtained from each library. DNA preparation was made from each library and the DNA was pooled together (named pBIB16).

The *Aspergillus* strain MT2425 (a *pyrG* minus strain, which gives small transformant-clones; when grown on the selection plates) was transformed with 1 microgram of the pBIB16 DNA and 10 microgram herring sperm DNA (carrier DNA) pr. 100 microliter protoplast using standard procedures.

The transformed protoplast were spread on selection plates (2 % maltose (inducing small morphology and lipase expression), 10mM NaNO₃, 1.2 M sorbitol, 2 % bacto agar, and standard salt solution.

After 5 days of growth, an overlay (containing 0.004 % brilliant green, 2.5 % olive oil, 1 % agar, 50 mM TRIS pH 7.5 treated with a mixer for 1 min. (Ultrathorax™ Type T25B, IKA Labortechnic, Germany)) was poured onto the *Aspergillus* transformant clones. The plates where incubated over night at room temperature.

Twenty of the clones having highest activity towards olive oil were inoculated in to 200 microliter YPM in a 96 well microtiter plate. After 4 days of growth at 34°C, the culture broths were assayed for lipase activity using pnp-valerate as described above.

The 6 transformants giving the highest activity in the lipase assay were inoculated in 5 ml YPM. DNA was isolated and transformed into the *E.coli* strain DH10B, thus rescuing the plasmid (as also described in WO 00/24883). Two pyrG variants were identified:
1) F96S; the plasmid was denoted **pENI2343**, and
2) T102N; the plasmid was denoted **pENI2344.**

Approx. 2 microgram of each of the plasmids pENI2155, pENI2343 and pENl2344 were transformed into an *Aspergillus* oryzae pyrG-minus mutant denoted Jal355, and an *Aspergillus nigerpyrG-minus* mutant denoted Mbin115, using standard procedures.

The transformed protoplasts were spread on selection plates (2 % maltose 10 mM NaNO₃, 1.2 M sorbitol, 2 % bacto agar, salt solution. After 4 days of growth, very poor sporulation was seen for the pENl2343 Jal355 transformants, and no transformants were seen for MBIN115 transformed with pENI2343.

6 independent transformants of each plasmid transformation were inoculated into 200 microliter 1*vogel, 2% maltose in a 96-well microtiter plate. After 4 days growth at 34°C, the culture broths were assayed for lipase activity. The results are given in the table below as relative lipase units with relative standard deviation, and are averages of the activity of the independent clones.

| | **JaI355** | **Mbin115** |
|---|---|---|
| pENI2155 (wt) | 48 ± 8% | 7 ± 14% |
| pENI2343 (F96S) | 49 ± 15% | No growth |
| pENI2344 (T102N) | 71 ± 13% | 80 ±11% |

The expression of lipase from the pENl2343 transformants was very high compared to the fungal biomass in the wells, which was very poor (less than 1/10 of the other transformants). An approx. 1.5-fold increase in lipase expression level is seen for the Jal355 transformants, and an approx. 11-fold increase is seen in the Mbin115 transformants; when comparing the pENI2155 transformants with the pEN12344 transformants.

Thus the pyrG T102N mutation leads to an increase in lipase expression, likely due to an increased plasmid copy number, which is selected for because of the unstable, less active OMP decarboxylase encoded by the selection gene *pyrG.*

### Example 3

In order to evaluate plasmid stability, a screen was set up to evaluate the percentage of spores containing a stably episomaly replicated plasmid (comprising a *pyrG* selection gene).

Two DNA libraries were constructed, the first library was cloned into a plasmid comprising the wildtype *pyrG* gene as selection gene, whereas the second library was cloned into a plasmid comprising a mutated *pyrG* gene which comprised a mutated Kozak region as described in Example1 and a T102N mutation as described in Example 2.

A spore suspension was made from each library and plated on to growth plates (2 % maltose 10 mM NaNO₃, 1.2 M sorbitol, 2 % bacto agar, salts, with or without 20 mM uridine). The plates were grown for 3 days at 37°C. Results are shown in the table below.

| **Selection gene** | **- uridine** | **+ uridine** | **% viable spores** |
|---|---|---|---|
| Wildtype *pyrG* | 11 | 83 | 13 |
| Mutant (Kozak/T102N) *pyrG* | 36 | 63 | 57 |

Evidently a much larger fraction of the spores contain a plasmid, when using the mutated (Kozak/T102N) *pyrG* gene.

## Claims

1. A method for isolating a recombinant polypeptide of interest, the method comprising the steps of:
a) providing a polynucleotide library derived from an organism capable of producing one or more polypeptides of interest, wherein the library was prepared in an expression cloning vector comprising at least the following elements:
i) a polynucleotide encoding a selectable marker in which the translation initiation start site of the marker-encoding sequence comprises the following sequence: 
-4
N YNN ATG YNN, SEQ ID NO:1,
 wherein "Y" in position -3 is a pyrimidin, "N" is any nucleotide, and the numerical designations are relative to the first nucleotide in the start-codon "ATG" of the marker;
ii) a fungal replication initiation sequence; and
iii) a polynucleotide comprising in sequential order: a promoter derived from a filementous fungal cell, a cloning-site into which the library is cloned, and a transcription terminator;
b) transforming a filamentous fungal host cell with the library;
c) culturing the transformed host cell obtained in (b) under conditions suitable for expression of the polynucleotide library; and
d) selecting a transformed host cell which produces the polypeptide of interest.

2. The method according to claim 1, wherein the organism of step (a) capable of producing one or more polypeptides of interest is a eukaryote.

3. The method according to claim 2, wherein the eukaryote is a filamentous fungus.

4. The method according to any of claims,1 - 3, wherein the sequence SEQ ID NO:1 comprises a Thymidin in the -3 position.

5. The method according to claim 4, wherein the sequence SEQ ID NO:1 further comprises a Thymidin in one or more of the positions-1,-2, and-4.

6. The method according to any of claims 1 - 5, wherein the selectable marker of step (i) is selected from the group of markers consisting of *amdS, argB, bar, hygB, niaD, pyrG, sC, and trpC.*

7. The method according to claim 6, wherein the selectable marker of step (i) is *pyrG* or a functional derivative thereof.

8. The method according to claim 7, wherein the selectable marker of step (i) is a functional derivative of *pyrG* which comprises a substitution of one or more amino acids.

9. The method according to anyof claims 1-8, wherein the fungal replication initiation sequence of step (ii) comprises the AMA1 sequence.

10. The method according to any of claims 1 - 9, wherein the filamentous fungal host cell is of the genus *Acremonium, Aspergillus, Coprinus, Fusarium, Humicola, Mucor, Myceliopthora, Neurospora, Penicillium, Thielavia, Tolypocladium* or *Trichoderma.*

11. The method according to claim 10, wherein the cell is of the species *Aspergillus oryzae, Aspergillus niger, Aspergillus nidulans, Coprinus cinereus, Fusarium oxysporum,* or *Trichoderma reesei.*

12. The method according to any of claims 1-11, wherein the polypeptide of interest is an enzyme, an enzyme variant, or a functional derivative thereof.

13. The method according to claim 12, wherein the enzyme or enzyme variant is an oxidoreductase, transferase, hydrolase, lyase, isomerase, or ligase.

14. The method according to claim 12 or 13, wherein the enzyme or enzyme variant is an aminopeptidase, amylase, carbohydrase, carboxypeptidase, catalase, cellulase, chitinase, cutinase, cyclodextrin glycosyltransferase, deoxyribonuclease, esterase, alpha-galactosidase, beta-galactosidase, glucoamylase, alpha-glucosidase, beta-glucosidase, invertase, laccase, lipase, mannosidase, mutanase, oxidase, a pectinolytic enzyme, peroxidase, phytase, polyphenoloxidase; proteolytic enzyme, ribonuclease, transglutaminase, or xylanase.

15. The method according to any of claims 1-11, wherein the polypeptide of interest is a hormone or hormone variant or a functional derivative thereof, a receptor or receptor variant or a functional derivative thereof, an antibody or antibody variant or a functional derivative thereof, or a reporter.

## Patentansprüche

1. Verfahren zum Isolieren eines rekombinanten Polypeptids von Interesse, wobei das Verfahren die Schritte umfasst:
a) Bereitstellen einer Polynukleotidbibliothek, abgeleitet aus einem Organismus, der zum Erzeugen eines oder mehrerer Polypeptide von Interesse fähig ist, wobei die Bibliothek in einem Expressionsklonierungsvektor hergestellt wurde, der mindestens die folgenden Elemente umfasst:
i) ein Polynukleotid, das einen Selektionsmarker kodiert, in welchem die Translationsinitiationsstartstelle der Marker-kodierenden Sequenz die folgende Sequenz umfasst: 
-4
N YNN ATG YNN,   SEQ ID NO: 1
 wobei "Y" in Position -3 ein Pyrimidin ist, "N" ein beliebiges Nukleotid, und die numerischen Bezeichnungen sich auf das erste Nukleotid in dem Startkodon "ATG" des Markers beziehen;
ii) eine Pilz-Replikationsinitiationssequenz; und
iii) ein Polynukleotid, umfassend in sequenzieller Reihenfolge: einen Promotor, abgeleitet aus einer filamentösen Pilzzelle, eine Klonierungsstelle, in welche die Bibliothek kloniert wird, und einen Transkriptionsterminator;
b) Transformieren einer filamentösen Pilzwirtszelle mit der Bibliothek;
c) Kultivieren der in (b) erhaltenen transformierten Wirtszelle unter Bedingungen, die für die Expression der Polynukleotidbibliothek geeignet sind; und
d) Auswählen einer transformierten Wirtszelle, welche das Polypeptid von Interesse erzeugt.

2. Verfahren nach Anspruch 1, wobei der zum Erzeugen eines oder mehrerer Polypeptide von Interesse fähige Organismus aus Schritt (a) ein Eukaryot ist.

3. Verfahren nach Anspruch 2, wobei der Eukaryot ein filamentöser Pilz ist.

4. Verfahren nach einem beliebigen der Ansprüche 1-3, wobei die Sequenz SEQ ID NO: 1 ein Thymidin in der -3-Position umfasst.

5. Verfahren nach Anspruch 4, wobei die Sequenz SEQ ID NO: 1 weiterhin ein Thymidin in einer oder mehreren der Positionen -1, -2 und -4 umfasst.

6. Verfahren nach einem beliebigen der Ansprüche 1-5, wobei der Selektionsmarker des Schrittes (i) ausgewählt ist aus der Gruppe von Markern, bestehend aus *amdS, argB, bar, hygB, niaD, pyrG, sC* und *trpc.*

7. Verfahren nach Anspruch 6, wobei der Selektionsmarker des Schrittes (i) *pyrG* oder ein funktionelles Derivat davon ist.

8. Verfahren nach Anspruch 7, wobei der Selektionsmarker des Schrittes (i) ein funktionelles Derivat von *pyrG* ist, welcher eine Substitution von einer oder mehreren Aminosäuren umfasst.

9. Verfahren nach einem beliebigen der Ansprüche 1-8, wobei die Pilzreplikationsinitiationssequenz des Schrittes (ii) die AMA1-Sequenz umfasst.

10. Verfahren nach einem beliebigen der Ansprüche 1-9, wobei die filamentöse Pilzwirtszelle aus der Gattung *Acremonium, Aspergillus, Coprinus, Fusarium, Humicola, Mucor, Myceliopthora, Neurospora, Penicillium, Thielavia, Tolypocladium* oder *Trichoderma* ist.

11. Verfahren nach Anspruch 10, wobei die Zelle von der Spezies *Aspergillus oryzae, Aspergillus niger, Aspergillus nidulans, Coprinus cinereus, Fusarium oxysporum* oder *Trichoderma reesei* ist.

12. Verfahren nach einem beliebigen der Ansprüche 1-11, wobei das Polypeptid von Interesse ein Enzym, eine Enzymvariante oder ein funktionelles Derivat davon ist.

13. Verfahren nach Anspruch 12, wobei das Enzym oder die Enzymvariante eine Oxidoreduktase, Transferase, Hydrolase, Lyase, Isomerase oder Ligase ist.

14. Verfahren nach Anspruch 12 oder 13, wobei das Enzym oder die Enzymvariante eine Aminopeptidase, Amylase, Carbohydrase, Carboxypeptidase, Catalase, Cellulase, Chitinase, Cutinase, Cyclodextringlycosyltransferase, Desoxyribonuklease, Esterase, Alpha-Galactosidase, Beta-Galactosidase, Glucoamylase, Alpha-Glucosidase, Beta-Glucosidase, Invertase, Laccase, Lipase, Mannosidase, Mutanase, Oxidase, ein pektinolytisches Enzym, Peroxidase, Phytase, Polyphenoloxidase, proteolytisches Enzym, Ribonuklease, Transglutaminase oder Xylanase ist.

15. Verfahren nach einem beliebigen der Ansprüche 1-11, wobei das Polypeptid von Interesse ein Hormon oder Hormonvariante oder ein funktionelles Derivat davon, ein Rezeptor oder eine Rezeptorvariante oder ein funktionelles Derivat davon, ein Antikörper oder eine Antikörpervariante oder ein funktionelles Derivat davon oder ein Reporter ist.

## Revendications

1. Méthode pour isoler un polypeptide recombinant d'intérêt, la méthode comprenant les étapes de :
a) fournir une banque de polynucléotides dérivée d'un organisme capable de produire un ou plusieurs polypeptides d'intérêt, dans laquelle la banque a été préparée dans un vecteur de clonage d'expression comprenant au moins les éléments suivants :
i. un polynucléotide codant un marqueur sélectionnable dans lequel le site de départ de l'initiation de la traduction de la séquence codant le marqueur comprend la séquence suivante : 
-4
N YNN ATG YNN, SEQ ID NO:1,
 dans laquelle "Y" en position -3 est une pyrimidine, "N" est n'importe quel nucléotide, et les désignations numériques sont relatives au premier nucléotide dans le codon de départ "ATG" du marqueur ;
ii. une séquence d'initiation de réplication fongique ; et
iii. un polynucléotide comprenant dans cet ordre : un promoteur dérivé d'une cellule fongique filamenteuse, un site de clonage dans lequel la banque est clonée, et un terminateur de transcription ;
b) transformer une cellule hôte fongique filamenteuse avec la banque ;
c) cultiver la cellule hôte transformée obtenue en (b) dans des conditions adaptées pour l'expression de la banque de polynucléotides ; et
d) sélectionner une cellule hôte transformée qui produit le polypeptide d'intérêt.

2. Méthode selon la revendication 1, dans laquelle l'organisme de l'étape a) capable de produire un ou plusieurs polypeptides d'intérêt est un eucaryote.

3. Méthode selon la revendication 2, dans laquelle l'eucaryote est un champignon filamenteux.

4. Méthode selon l'une quelconque des revendications 1 à 3, dans laquelle la séquence SEQ ID NO:1 comprend une thymidine en position -3.

5. Méthode selon la revendication 4, dans laquelle la séquence SEQ ID NO:1 comprend également une thymidine dans l'une ou plusieurs des positions -1, -2 et - 4.

6. Méthode selon l'une quelconque des revendications 1 à 5, dans laquelle le marqueur sélectionnable de l'étape i. est sélectionné dans le groupe de marqueurs constitué de *amdS, argB, bar, hygB, niaD, pyrG, sC,* et *trpc.*

7. Méthode selon la revendication 6, dans laquelle le marqueur sélectionnable de l'étape i. est *pyrG* ou un de ses dérivés fonctionnels.

8. Méthode selon la revendication 7, dans laquelle le marqueur sélectionnable de l'étape i. est un dérivé fonctionnel de *pyrG* qui comprend une substitution d'un ou plusieurs acide(s) aminé(s).

9. Méthode selon l'une quelconque des revendications 1 à 8, dans laquelle la séquence d'initiation de la réplication fongique de l'étape ii. comprend la séquence AMA1.

10. Méthode selon l'une quelconque des revendications 1 à 9, dans laquelle la cellule hôte fongique filamenteuse est du genre *Acremonium, Aspergillus, Coprinus, Fusarium, Humicola, Mucor, Myceliopthora, Neurospora, Penicillium, Thielavia, Tolypocladium* ou *Trichoderma.*

11. Méthode selon la revendication 10, dans laquelle la cellule est de l'espèce *Aspergillus oryzae, Aspergillus niger, Aspergillus nidulans, Coprinus cinereus, Fusarium oxysporum,* ou *Trichoderma reesei.*

12. Méthode selon l'une quelconque des revendications 1 à 11, dans laquelle le polypeptide d'intérêt est une enzyme, un variant d'enzyme ou un de leurs dérivés fonctionnels.

13. Méthode selon la revendication 12, dans laquelle l'enzyme ou le variant d'enzyme est une oxydoréductase, transférase, hydrolase, lyase, isomérase, ou ligase.

14. Méthode selon la revendication 12 ou 13, dans laquelle l'enzyme ou le variant d'enzyme est une aminopeptidase, amylase, carbohydrase, carboxypeptidase, catalase, cellulase, chitinase, cutinase, cyclodextrine glycosyltransférase, désoxyribonucléase, estérase, alpha-galactosidase, beta-galactosidase, glucoamylase, alpha-gucosidase, beta-glucosidase, invertase, laccase, lipase, mannosidase, mutanase, oxydase, une enzyme pectinolytique, peroxydase, phytase, polyphénoloxydase, enzyme protéolytique, ribonucléase, transglutaminase, ou xylanase.

15. Méthode selon l'une quelconque des revendications 1 à 11, dans laquelle le polypeptide d'intérêt est une hormone ou un variant d'hormone ou un de leurs dérivés fonctionnels, un récepteur ou un variant de récepteur ou un de leurs dérivés fonctionnels, un anticorps ou un variant d'anticorps ou un de leurs dérivés fonctionnels, ou un rapporteur.
